# EUROPÄISCHE PATENTANMELDUNG

(11) **EP 0 575 719 A1**
(43) Veröffentlichungstag der Anmeldung: **29.12.1993**
(21) Anmeldenummer: 93106212.9
(22) Anmeldetag: 16.04.1993
(51) Int. Cl.: A61F 2/06

(54) **Vorrichtung zur Korrektur der Lage eines Stents**

(30) Priorität: 20.06.1992 DE 4220295
(71) Anmelder: ANGIOMED AG, D-76227 Karlsruhe (DE)
(72) Erfinder: Lindenberg, Josef, W-7500 Karlsruhe 41 (DE); Schnepp-Pesch, Wolfram, W-7500 Karlsruhe 41 (DE)
(74) Vertreter: Dipl.-Ing. Heiner Lichti Dipl.-Phys. Dr.rer.nat. Jost Lempert Dipl.-Ing. Hartmut Lasch

(57) **Zusammenfassung**

Es ist bekannt zum Aufweiten bzw. Offenhalten einer Stenose, sei es in Blutgefäßen, im Harnleiter, in der Harnröhre oder dergleich kurze Stents zu legen, die Durchbrechungen aufweisen und beispielsweise aus gestrickten oder gewirkten Maschendraht bestehen. Es kann vorkommen, daß die Stents von vornherein nicht richtig positioniert werden oder sich verschieben. Beispielsweise können sie in der Harnröhre im Bereich des Urethra Sphinkter zu liegen gelangen, so daß dieser nicht mehr wirken könnte.

Die Erfindung schlägt daher in der Vorrichtung zur Korrektur der Lage eines solchen Stents mit einem Führungsteil vor, welche gekennzeichnet ist durch ein an seinem vorderen Ende (4) mit radial aufstellbaren Drahtabschnitten (9) versehem länglichen Drahtteil (2).

## Beschreibung

Die Erfindung betrifft ein Vorrichtung zur Korrektur der Lage eines Stents in einem Gefäß oder dergleichen, wie eines Stents zum Aufweiten einer Stenose oder dergleichen, mit mindestens einem Führungsteil.

Zum Offenhalten von Stenosen oder dergleichen in Blutgefäßen oder sonstigen Hohlkörpern des menschlichen Körpers, wie Ureter, Urethra oder dergleichen werden kurze Stents verwendet, die im Bereich der Stenose eingelegt werden und durch ihre Eigenstabilität diese aufhalten sollen. Es werden vorzugsweise Stents aus Draht eingesetzt, die entweder mittels eines Ballonkatheters in ihre radialexpandierte Stellung aufgeweitet werden oder aber bei Ausbildung aus Material einer Gedächtnislegierung nach dem Einführen aufgrund der Körperwärme sich selbständig radial vorgeprägte aufgeweitete Stellung aufweiten. Es kann vorkommen, daß die Positionierung eines solchen Stents nicht in der gewünschten Weise erfolgt. Es kann auch vorkommen, daß ein Stent sich trotz der Aufweitung aus irgendwelchen Gründen verschiebt. Wenn ein Stent beispielsweise in der Harnröhre in den Bereich des Urethrasphinkters gelangt, so kann dieser seine Funktion nicht mehr erfüllen, was zur Inkontinenz führen kann. In einem solchen Falle ist es wünschenswert, entweder sogleich ein Nachlegen des Stents durchzuführen, wenn die ursprüngliche Position nicht gewünscht ist, oder auch später die axiale Lage des Stents in dem Körpergefäß zu korrigieren. Gleiches gilt für Stents in anderen Organen, wie Gallen- oder auch Femoralstents.

Der Erfindung liegt daher die Aufgabe zugrunde, eine Vorrichtung zu schaffen, die eine Korrektur der Position eines derartigen Stents erlaubt.

Erfindungsgemäß wird die genannte Aufgabe bei einer Vorrichtung der eingangs genannten Art gelöst durch ein an seinem vorderen Ende mit radial aufstellbaren Drahtabschnitten versehenes längliches Drahtteil.

Das mit den radial aufstellbaren Drahtabschnitten versehene längliche Drahtteil wird durch ein Führungsteil, wie Zystoskop, das gegebenfalls einen zweiten Arbeitskanal aufweist, einen Katheter, ein Rohrteil, ein Hohlstilett oder dergleichen sei es in Blutgefäßen, im Harnleiter oder dergleichen bis in den Bereich des Stents eingeführt, und zwar wenn der Stent weiter vorzuschieben ist, bis kurz vor den stent, wenn der Stent zurückzuziehen ist, bis hinter den Stent, oder aber auch lediglich in den vom Stent überdeckten Bereich. Anschließend erfolgt die radiale Aufweitung der radial aufweitbaren Drahtabschnitte, sei es, daß diese derart vorgeprägt sind, daß sie sich nach Heraustreten aus dem Führungsteil, wenn dieses zurückgezogen wird, selbständig radial aufstellen, sei es, daß die radial aufweitbaren Drahtabschnitte durch eine geeignete Einrichtung, wie einen Zugdraht, aufgeweitet werden können, bis ihre am weitesten radial nach außen ragenden Bereiche vor oder hinter dem Stent oder in dessen Mittelbereich an diesem angreifen können. Durch Vorschieben oder Zurückziehen des Drahtteils kann dann die Lage des Stents verändert werden.

Gemäß einer bevorzugten Ausgestaltung ist vorgesehen, daß die Drahtabschnitte bei Entlastung selbstaufstellende Drahtabschnitte sind, wobei insbesondere die Drahtabschnitte einstückig mit dem länglichen Drahtteil ausgebildet sind. In einem solchen Fall besteht der Drahtabschnitt vorzugsweise aus ineinandergebundenen einzelnen Drahtlitzen, die sich im Bereich der aufstellbaren Drahtabschnitte teil, an ihrem vorderen freien Ende aber wieder zusammengefaßt sind. Die Drahtabschnitte weisen eine Vorprägung derart auf, daß sie sich bei Freigabe von von außen wirkenden Kräften radial aufstellen können. Eine alternative Ausgestaltung sieht vor, daß das Drahtteil einen durch einen Hohldraht geführten Zugdraht aufweist, der am vorderen Ende der Drahtabschnitte an diesem angreift, während diese an ihrem rückwärtigen Ende mit dem vorderen Ende des Hohldrahtes verbunden sind, wobei auch die Drahtabschnitte einstückig mit dem Hohldraht ausgebildet sind. Bei einer solchen Ausgestaltung kann das Aufstellen der aufstellbaren Drahtabschnitte durch Ziehen am Zugdraht erfolgen.

Um ein besseres Angreifen der radial aufweitbaren Drahtabschnitte am Stent zu ermöglichen, sehen weitere bevorzugte Ausgestaltungen vor, daß die radial aufstellbaren Drahtabschnitte in ihrem radial am weitestens nach außen ragenden Bereich mit Ecken versehen sind und/oder die radial aufstellbaren Drahtabschnitte an ihren radial am weitesten nach außen ragenden Bereichen mit Haken versehen sind. In alternativer Ausgestaltung kann vorgesehen sein, daß die radial aufstellbaren Drahtabschnitte an ihren radial am weitesten nach außen ragenden Bereichen mit Verdickungen versehen sind, wobei insbesondere die Verdickungen kleine Kugeln sind.

Während das Führungsrohr grundsätzlich als Metallrohr ausgebildet sein kann, kann es an seinem vorderen Ende mit flachen Kanten versehen und damit als Hohlstilett ausgebildet sein. Gegebenenfalls wird es perkutan unter Zuhilfenahme eines Innenstiletts eingeführt. Das Führungsteil kann auch flexibel, beispielsweise in Form eines Katheters ausgebildet sein.

Während die radial aufstellbaren Drahtabschnitte grundsätzlich ein Körbchen bilden, können sie auch schraubenwendelförmig ausgebildet sein, wobei auch in diesem Falle die Wendel im Querschnitt oder in der Projektion nicht lediglich einen kreisförmigen, ovalen oder elliptischen Querschnitt aufweisen muß, sondern in Projektion auch kantig ausgebildet sein kann.

Gemäß einer äußerst bevorzugten Ausgestaltung der Erfindung ist vorgesehen, daß die radial aufstellbaren Drahtabschnitte am länglichen Drahtabschnitt (2) befestigt sind, wobei insbesondere die aufstellbaren Drahtabschnitte im länglichen Drahtabschnitt angelötet sind. Während die radial aufstellbaren Drahtabschnitte grundsätzlich aus Stahl, wie Federstahl, bestehen können und von einem Führungsrohr oder -schlauch in ihrer aneinander anliegenden Einführposition gehalten werden, wobei sie sich beim Vorschieben aus dem Führungsrohr oder -schlauch heraus aufgrund ihrer Vorspannung radial aufstellen, so daß die radial aufstellbaren Drahtabschnitte sich in entlastetem Zustand in ihre radial aufgeweitete Stellung bewegen, sieht eine bevorzugte Ausgestaltung vor, daß die radial aufstellbaren Drahtabschnitte aus einer Nickel-Titan-Legierung (Nitinol) bestehen. Auch die Verwendung eines solchen Materials ermöglicht vorstehend Beschriebenes. In bevorzugter Weiterbildung ist dann aber vorgesehen, daß sich die radial aufstellbaren Drahtabschnitte in einer Tieftemperaturstellung im wesentlichen achsparallel ausrichten und in einer Hochtemperaturstellung sich radial weiter nach außen erstrecken.

Während die Drahtabschnitte grundsätzlich ein körbchenartiges Gebilde bilden können, wobei sie mit ihrem vordersten Ende zueinander geführt und miteinander verbunden sind und sich in Zwischenbereichen radial aufstellen, sieht eine äußerst bevorzugte Ausgestaltung vor, daß in radial nach außen gestellter Position radial am weitesten nach außen ragende Bereiche der radial aufstellbaren Drahtabschnitte deren freie Enden sind, wobei insbesondere in aufgeweiteter Stellung der radial aufstellbaren Drahtabschnitte sich am weitesten radial nach außen erstreckende Bereiche etwa senkrecht zur Symmetrieachse der Vorrichtung stehen. Zur Vermeidung einer Verletzungsgefahr in diesem Falle zeichnet sich eine bevorzugte Weiterbildung dadurch aus, daß die freien Enden mit Abstumpfungen, beispielsweise in Form von kugelförmigen Enden, versehen sind. Um insbesondere hier in der Einführposition, bei der die Drahtabschnitte in einem Führungsrohr oder -schlauch eng nebeneinander angeordnet sind, möglichst wenig radialen Platzbedarf zu benötigen - und damit dünne Führungsrohre oder -schläuche verwenden zu können -, sieht eine äußerst bevorzugte Ausgestaltung vor, daß die radial aufstellbaren Drahtabschnitte unterschiedliche Längen aufweisen. Die Enden, insbesondere wenn diese mit Kügelchen oder dergleichen versehen sind, befinden sich dann in der Einführposition nicht in einer gemeinsamen Radialebene, so daß die Kügelchen nicht nebeneinander liegen, sondern sind in axialer Richtung zueinander versetzt, so daß auch die Kügelchen an den verschiedenen radial aufstellbaren Drahtenden radial hintereinander angeordnet sind.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnungen im einzelnen erläutert sind. Dabei zeigt bzw. zeigen:
- Figur 1: eine erste Ausführungsform der erfindungsgemäßen Vorrichtung zur Korrektur der Lage eines Stents mit einem Zugdraht;
- Figur 2: eine weitere Ausgestaltung der erfindungsgemäßen Vorrichtung zur Korrektur der Lage eines Stents mit einem Hohlstilett als Führungsrohr;
- Figur 3: weitere gegebenenfalls zu einer erfindungsgemäßen Vorrichtung zur Korrektur der Lage eines Stents gehörende Elemente mit Führungsrohr an dem zu legenden Stent sowie einem Schieber zum Ausschieben desselben aus dem Führungsrohr;
- Figur 4a-4c: Ausgestaltungen von an den aufstellbaren Drahtabschnitten ausgebildeten bzw. vorgesehenen Elementen zum Angreifen am Stent in Form von am aufstellbaren Drahtabschnitt zusätzlich vorgesehenen Haken (4a), bzw. an den Drahtabschnitten selbst ausgebildeten Haken (4b) bzw. an den aufstellbaren Drahtabschnitten angeordneten kugelförmigen Erweiterungen;
- Figur 5a-5b: weitere Ausgestaltungen der aufstellbaren Drahtabschnitte einer erfindungsgemäßen Vorrichtung mit (5a) einer Schraubenwendel mit kreisförmigen Windungen und (5b) einer Schraubenwendel mit mehreckförmigen Windungen;
- Figuren 6a-c: eine weitere bevorzugte Ausgestaltung der erfindungsgemäßen Vorrichtung, bei der im radial aufgestellten Funktionszustand die freien Enden der aufstellbaren Drahtabschnitte sich radial am weitesten nach außen erstrecken, und zwar in Richtungen enden, die im wesentlichen senkrecht zur Achse des länglichen Drahtteils verlaufen, wobei die freien Enden mit abgerundeten Kügelchen versehen sind (Fig. 6a, 6c) und unterschiedliche Längen aufweisen (Fig. 6c); und
- Figur 7: eine Betätigungseinrichtung für eine erfindungsgemäße Vorrichtung nach einer der vorstehend genannten Ausgestaltungen.

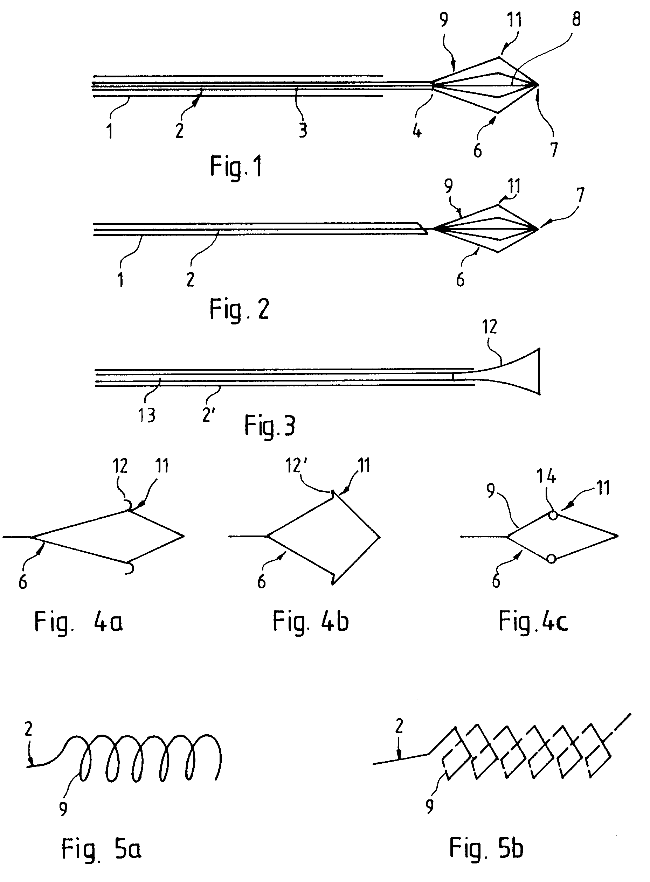

Die erfindungsgemäße Vorrichtung zur Korrektur der Lage eines in ein Gefäß oder dergleichen gelegten Stents weist zunächst ein Führungsteil 1 in Form eines Führungsrohres oder Führungsschlauches oder auch eines Stiletts (Figur 2) auf. Weiterhin weist die erfindungsgemäße Vorrichtung ein längliches Drahtteil 2 auf. Das Drahtteil 2 hat in der in Figur 1 dargestellten Ausführungsform ein Hohldrahtelement 3 beispielsweise aus schraubenförmig gewundenem Draht, dessen einzelne Windungen aneinander liegen. Am vorderen Ende 4 des Hohldrahts 3 ist entweder ein Körbchen 6 am Hohldrahtteil befestigt oder aber Einzeldrähte des Hohldrahts 3 sind nicht mehr gewendelt, sondern in einer Längsebene bis zum vorderen Ende 7 des gesamten Drahtteils 2 geführt. Dort sind sie mit dem vorderen Ende eines Zugdrahts 8 verbunden, der durch das Hohldrahtteil 3 bis zu dessen rückwärtigem Ende und aus diesem heraus (nicht dargestellt) geführt ist. Die Drahtabschnitte 9 des Körbchens 6 können vorgeprägte Ecken oder Kanten 11 aufweisen. Ohne Belastung liegen die Drahtabschnitte 9 eng aneinander, so daß das gesamte Drahtteil 2 durch das Führungsteil 1 bewegt werden kann. Wenn das Körbchen 6 mit den Drahtabschnitten 9 im Bereich eines gelegten Stents 12 angeordnet ist, kann es durch Ziehen am Zugdraht 8 in die in Figur 1 dargestellte radial aufgeweitete Stellung expandiert werden. Die Ecken 11 greifen dann wie nach axialer Positionierung entweder vor oder hinter den Stent 12 oder aber im Inneren an diesem, beispielsweise seinen Maschen oder Windungen, an. Durch Verschieben des Drahtteils 2 kann dann der Stent 12 ebenfalls axial hin und her bewegt und in geeigneter Weise besser positioniert werden.

Bei der Figur 2 ist das Führungsteil 2 als Hohlstilett ausgebildet. Hierdurch kann beispielsweise eine Korrektur der Lage eines Stents perkutan erfolgen, wie beispielsweise bei einem Gallen- oder auch Femoralstent. Das Hohlstilett in Figur 2 kann auch bei einer ansonsten entsprechend der Figur 1 ausgebildeten Vorrichtung eingesetzt werden, während das Führungsteil der Figur 1, sei es als Rohr oder als Schlauch, auch bei einer Vorrichtung gemäß der Ausbildung der Figur 2 eingesetzt werden kann, wie sie im folgenden beschrieben wird.

Bei der Ausgestaltung der Figur 2 kann das längliche Drahtteil 2 ein Einzeldraht oder auch ein gewendelter Draht gegebenfalls aus mehreren Einzellitzen sein, die dann wiederum ein Körbchen 6 bilden, welches aber auch separat an dem Drahtteil 2 angesetzt sein kann. Bei der Ausgestaltung der Figur 2 sind die Drahtabschnitte 9 des Körbchens 6 in der dargestellten radial aufgeweiteten Stellung vorgespannt bzw. vorgeprägt, so daß sich das Körbchen 6 ohne Einwirkung äußerer Kräfte in diese radial aufgeweitete Position aufstellt. Die Einzeldrähte 9 sind am vorderen freien Ende 7 des Körbchens 6 miteinander verbunden. Wird das Körbchen 6 in das Führungsteil 1 hineingezogen, so wird es durch dieses radial zusammengepreßt. Zunächst erfolgt eine Einführung des Führungsteils 1 bis in den Bereich des in seiner Lage zu korrigierenden Stents. Das Drahtteil 2 mit dem im Führungsteil 1 befindlichen radial zusammengepreßten Körbchen 6 wird dann weiter vorgeschoben, wodurch das Körbchen 6 freigegeben wird und sich radial in die in Figur 2 dargestellte Position aufweiten kann. Es greift damit wiederum mit seinen Kanten 11 nach axialer Positonierung vor, hinter oder im Bereich des Stents 12 an, so daß dieser durch Ziehen oder Schieben am Drahtteil 2 in seiner Position korrigiert werden kann.

Weitere Elemente der erfindungsgemäßen Vorrichtung können neben dem schon erwähnten Stent 12 ein Führungsteil 2', das dem Führungsteil 2 entsprechen kann sowie ein Schieber 13 sein, der durch das Führungsteil 2' hindurchgeschoben wird und den in diesem befindlichen Stent aus dem Führungsteil 2' herausschieben kann. Wenn dann noch eine Korrigierung der Axiallage des Stents 12 erforderlich ist, so kann nach Entfernen des Schiebers 13 ein Drahtteil 2 gemäß der Figuren 1 oder 2 durch das Führungsteil 2 eingeschoben werden, bis es am gelegten Stent 12 angreifen kann, um diesen dann zu positionieren.

Während in den Figuren 1 und 2 die Drahtabschnitte 9 des Körbchens 6 lediglich mit Ecken oder Kanten 11 versehen sind, können in den Bereichen der Ecken oder Kanten 11 auch Häkchen 12 vorgesehen sein, wie sie in Figur 4 dargestellt sind. Weiterhin können die Ecken oder Kanten 11 auch zu Haken 12' geformt sein, wie dies bei Figur 4 b der Fall ist. Als Angreifmittel zum Angreifen am Stent 12 bzw. Eingreifen in dessen Windungen oder Maschen können im Bereich der Kanten auch Erweiterungen an den Drahtteilen 9 beispielsweise in Form von Erweiterungen 14 an den Drahtteilen 9 beispielsweise in Form von Kügelchen vorgesehen sein.

Die Figur 5 zeigt eine andere Ausgestaltung der radial aufweitbaren Drahtabschnitte 9' bzw. 9'' in Form von Wendeln mit grundsätzlich Schraubenkontur. Während die Außenkontur bei dem Drahtabschnitt 9 zylindermantelförmig ist, dieser also einen kreisförmigen Querschnitt bzw. eine kreisförmige Axialprojektion aufweist, die allerdings auch beispielsweise elliptisch oder oval sein kann, weist der Wendeldrahtabschnitt 9'' der Figur 5 b mehrere Ecken oder Kanten 11 auf, und damit beispielsweise einen vier- oder mehreckigen Querschnitt bzw. eine entsprechende Axialprojektion. Derartige Ausgestaltungen werden insbesondere vorgesehen, wenn die Drahtabschnitte 9' bzw. 9'' im Bereich des Stents 12 selbst, also nicht vor oder hinter diesem, angreifen sollen. Die Ecken 11 greifen in diesem Falle wieder gut in die Maschen des Stents 12 ein, so daß dieser durch Schieben oder Ziehen optimal positioniert werden kann. Die Kanten 11 können entsprechend der Figuren 4a bis 4c weiter ausgebildet sein.

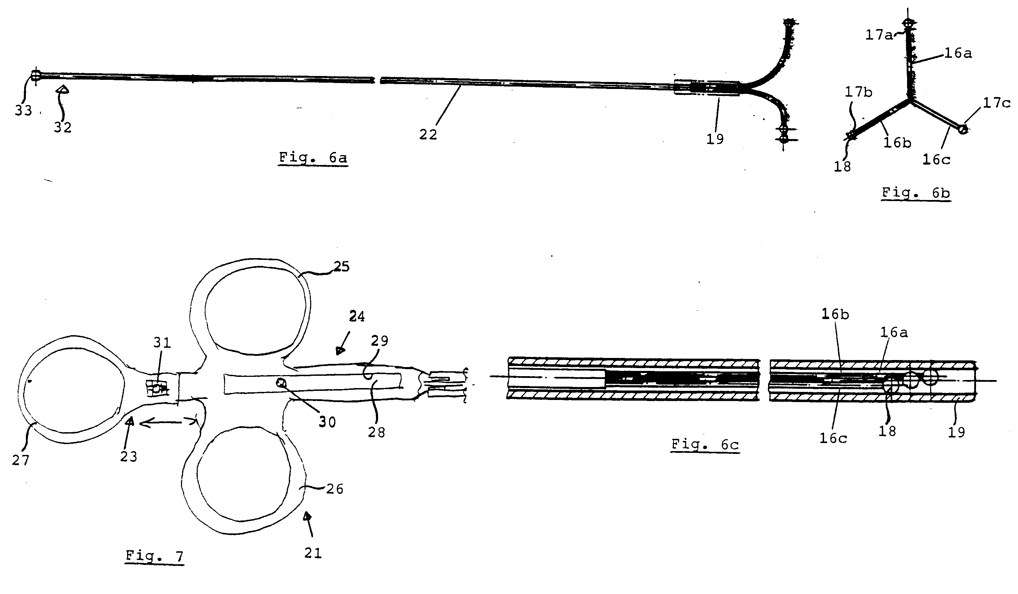

Die Figuren 6a-6c zeigen eine weitere erfindungsgemäße Ausgestaltung, bei der insbesondere die radial aufstellbaren Drahtabschnitte 16a, 16b, 16c aus einer Nickel-Titan-Legierung wie Nitinol bestehen und mit Formgedächtnis versehen sind. Auch der längliche Drahtteil 22 kann aus einer entsprechenden Legierung bestehen, wobei ihm keine Formgedächtniskontur aufgeprägt ist.

Zur Vermeidung von Verletzungen sind die aufstellbaren Drahtabschnitte 16a-16c an ihren freien Enden 17a-17c mit kleinen Kügelchen 18 versehen. Die erwünschte Abstumpfung oder Abrundung der freien Enden 17a-17c kann auch in anderer Weise erzielt werden.

In ihrer Tieftemperaturstellung, wie bei 0°, erstrecken die freien Drahtabschnitte 16a-16c sich parallel zueinander, wie dies der Figur 6c zu entnehmen ist. Sie weisen, wie dies den Figuren 6a-6c zu entnehmen ist, unterschiedliche Länge auf, so daß die Kügelchen 18 der einzelnen Drahtabschnitte 16a-16c sich in der Tieftemperaturstellung derselben an unterschiedlichen axialen Positionen befinden und nicht radial nebeneinander angeordnet sind, so daß das Innenlumen des Einführkatheters oder -rohres 19 geringer sein kann, als es der Fall wäre, wenn die drei Kugeln 18 sich in einer Radialebene befänden.

In der Hochtemperaturstellung des Nickel-Titan-Materials der aufstellbaren Drahtabschnitte 16a-16c, die unter 35 °C erreicht sein muß, wobei die Übergangstemperatur üblicherweise in einem Bereich zwischen 10 und 30° liegt, erstrecken die aufstellbaren Drahtabschnitte 16a-16c sich bogenförmig radial nach außen, wobei ihre freien Enden 17a-17c zur Achse des länglichen Drahtteils 22 einen Winkel von etwa 9° einschließen, wie dies insbesondere der Figur 6a deutlich zu entnehmen ist.

Zur Korrektur eines Stents wird die erfindungsgemäße Vorrichtung mit dem Führungsrohr 19 und in diesem liegenden länglichen Drahtteil 22 mit aufstellbaren Drahtabschnitten 16a-16c bis in den Bereich des vorher eingeführten Stents oder der Endoprothese eingeführt. Der längliche Drahtteil 22 wird vorgeschoben, bis die aufstellbaren Drahtabschnitte 16a-16c aus dem Einführschlauch oder -rohr 19 austreten und sich in die in den Figuren 6a und 6b gezeigte Stellung radial aufstellen, sei es unter von vornherein gegebener Vorspannung, sei es aufgrund ihrer in dieser Form gegebenen Hochtemperaturstellung. Die freien Enden 17a-17c mit den Kügelchen 18 greifen in Zwischenräume des Stents oder der Endoprothese, die aus Draht gebildet sein, insbesondere gestrickt sein kann, oder aus mit Druchbrüchen versehenem Flachmaterial besteht, so daß durch Vorschieben und Zurückziehen des länglichen Drahtteils 22 die Axialposition des Stents bzw. der Endoprothese korrigiert werden kann.

Zum Vorschieben des länglichen Drahtteils 22 im Führungsrohr oder -schlauch 19 ist vorzugsweise eine Betätigungseinrichtung 21 vorgesehen, wie diese in der Figur 7 dargestellt ist. Die Betätigungseinrichtung 21 besteht aus zwei Griffteilen 23, 24, die axial relativ zueinander bewegbar sind. Das Griffteil 24 weist zwei Greifringe 25, 26 auf, in die mit Zeige- und Mittelfinger eingegriffen wird, während das Griffteil 23 ein Ringteil 27 aufweist, in welches der Daumen eingreift. Am Griffteil 24 ist der Führungskatheter oder -schlauch 19 fest angebracht. Ein Axialansatz 28 des Griffteils 23 ragt in das Griffteil 24 und ist durch eine Schlitzführung 29 in diesem und einen Nocken 30 am Axialteil 28 begrenzt axial beweglich.

Das längliche Drahtteil 22 ist am Griffteil 23 festgelegt, beispielsweise mittels eines mit Federbeinchen 31 versehenen steckteils, wobei die Federteilchen 31 eine am rückwärtigen Ende 32 des länglichen Drahtteils 22 angebrachte Kugel 33 umgreifen, die einen größeren Durchmesser aufweist als das längliche Drahtteil 22 selbst. Derart wird das längliche Drahtteil 22 und mit ihm die aufstellbaren Drahtabschnitte 16a-16c sicher im Griffteil 23 gehalten. Die Figur 7 zeigt die vorgeschobene Betriebsstellung der erfindungsgemäßen Vorrichtung, bei der die aufstellbaren Drahtabschnitte 16a-16c aus dem Schlauch bzw. Rohr 19 herausragen (Figuren 6a und 6b). Bei der Einführstellung sind Griffteil 24 und Griffteil 23 axial auseinandergezogen, und zwar, bis der Nocken 30 am rückwärtigen (linken) Ende des Führungsschlitzes 29 des Griffteils 24 anschlägt. In dieser Einführposition befinden sich die aufstellbaren Drahtabschnitte 16a-16c in der in Figur 6 dargestellten Stellung innerhalb des Führungsschlauches oder -rohres 19.

Während die Ausgestaltung der Betätigungseinrichtung 21 nach der Figur 7 unter Bezugnahme auf eine ansonsten entsprechend den Figuren 6a-6c ausgestaltete Vorrichtung erläutert wurde, kann die Betätigungseinrichtung in gleicher Weise auch bei einer erfindungsgemäßen Vorrichtung mit ansonsten der Ausgestaltung nach den vorangehenden Figuren eingesetzt werden, wobei das Führungsteil 1 mit dem Griffteil 24 und das Drahtteil 22 mit dem Griffteil 23 fest verbunden ist.

## Patentansprüche

1. Vorrichtung zur Korrektur der Lage eines Stents in einem Gefäß oder dergleichen, wie eines Stents zum Aufweiten einer Stenose oder dergleichen, mit mindestens einem Führungsteil, gekennzeichnet durch ein an seinem vorderen Ende (4) mit radial aufstellbaren Drahtabschnitten (9, 9', 9'') versehenes längliches Drahtteil (2).

2. Vorrichtung nach Anspruch 1, dadurch gekennzeichnet, daß die Drahtabschnitte (9, 9', 9'') bei Entlastung selbstaufstellende Drahtabschnitte sind.

3. Vorrichtung nach Anspruch 2, dadurch gekennzeichnet, daß die Drahtabschnitte (9) einstückig mit dem länglichen Drahtteil (2) ausgebildet sind.

4. Vorrichtung nach einem der vorangehenden Ansprüche, dadurch gekennzeichnet, daß die radial aufstellbaren Drahtabschnitte am länglichen Drahtabschnitt (2) befestigt sind.

5. Vorrichtung nach Anspruch 4, dadurch gekennzeichnet, daß die aufstellbaren Drahtabschnitte am länglichen Drahtabschnitt (2) angelötet sind.

6. Vorrichtung nach einem der vorangehenden Ansprüche, dadurch gekennzeichnet, daß die radial aufstellbaren Drahtabschnitte sich in entlastetem Zustand in ihre radial aufgeweitete Stellung bewegen.

7. Vorrichtung nach einem der vorangehenden Ansprüche, dadurch gekennzeichnet, daß die radial aufstellbaren Drahtabschnitte aus einer Nickel-Titan-Legierung (Nitinol) bestehen.

8. Vorrichtung nach Anspruch 7, dadurch gekennzeichnet, daß sich die radial aufstellbaren Drahtabschnitte in einer Tieftemperaturstellung im wesentlichen achsparallel ausrichten und in einer Hochtemperaturstellung sich radial weiter nach außen erstrecken.

9. Vorrichtung nach einem der vorangehenden Ansprüche, dadurch gekennzeichnet, daß in radial nach außen gestellter Position radial am weitesten nach außen ragende Bereiche der radial aufstellbaren Drahtabschnitte deren freie Enden sind.

10. Vorrichtung nach einem der vorangehenden Ansprüche, dadurch gekennzeichnet, daß in aufgeweiteter Stellung der radial aufstellbaren Drahtabschnitte sich am weitesten radial nach außen erstreckende Bereiche etwa senkrecht zur Symmetrieebene der Vorrichtung stehen.

11. Vorrichtung nach einem der vorangehenden Ansprüche, dadurch gekennzeichnet, daß die radial aufstellbaren Drahtabschnitte unterschiedliche Längen aufweisen.

12. Vorrichtung nach einem der vorangehenden Ansprüche, dadurch gekennzeichnet, daß das Drahtteil (2) einen durch einen Hohldraht (3) geführten Zugdraht (8) aufweist, der am vorderen Ende (7) der Drahtabschnitte (9) an diesem angreift, während diese an ihrem rückwärtigen Ende mit dem vorderen Ende (4) des Hohldrahtes verbunden sind.

13. Vorrichtung nach Anspruch 12, dadurch gekennzeichnet, daß die Drahtabschnitte (9) einstückig mit dem Hohldraht (3) ausgebildet sind.

14. Vorrichtung nach einem der vorangehenden Ansprüche, dadurch gekennzeichnet, daß die radial aufstellbaren Drahtabschnitte (9, 9'') in ihrem radial am weitesten nach außen ragenden Bereich mit Ecken (11) versehen sind.

15. Vorrichtung nach einem der vorangehenden Ansprüche, dadurch gekennzeichnet, daß die radial aufstellbaren Drahtabschnitte (9, 9'') an ihrem radial am weitesten nach außen ragenden Bereich mit Haken (12, 12') versehen sind.

16. Vorrichtung nach einem der Ansprüche 1 bis 14, dadurch gekennzeichnet, daß die radial aufstellbaren Drahtabschnitte (9, 9', 9'') an ihren radial am weitesten nach außen ragenden Bereichen mit Verdickungen (14) versehen sind.

17. Vorrichtung nach Anspruch 16, dadurch gekennzeichnet, daß die Verdickungen kleine Kugeln sind.

18. Vorrichtung nach einem der vorangehenden Ansprüche, dadurch gekennzeichnet, daß das Führungsteil (2, 2') als Hohlstilett ausgebildet ist.

19. Vorrichtung nach einem der Ansprüche 1 bis 17, dadurch gekennzeichnet, daß das Führungsteil flexibel ist.

20. Vorrichtung nach einem der vorangehenden Ansprüche, gekennzeichnet durch einen Stent (12) zum Aufweiten einer Stenose und einen durch das Führungsteil (2, 2') hindurchschiebbaren Schieber (13) zum Herausschieben des Stents (12) aus dem Führungsteil (2, 2').
